# EUROPÄISCHE PATENTSCHRIFT

(11) **EP 1 195 369 B1**
(45) Veröffentlichungstag und Bekanntmachung des Hinweises auf die Patenterteilung: **16.02.2005**
(21) Anmeldenummer: 01123520.7
(22) Anmeldetag: 29.09.2001
(51) Int. Cl.: C07C 29/70, C07C 31/30

(54) **Verfahren zur katalytischen Herstellung von Alkalialkoholaten**
Process for the catalytic production of alkali metal alcoholates
Procédé pour la production catalytique d'alcoolats de métaux alcalins

(30) Priorität: 06.10.2000 DE 10050015
(43) Veröffentlichungstag der Anmeldung: 10.04.2002
(73) Patentinhaber: BASF AKTIENGESELLSCHAFT, 67056 Ludwigshafen (DE)
(72) Erfinder: Schierle-Arndt, Kerstin, Dr., 64673 Zwingenberg (DE); Sterzel, Hans-Josef, Dr., 67125 Dannstadt-Schauernheim (DE); Schläfer, Dieter, Dr., 67071 Ludwigshafen (DE); Guth, Josef, 67251 Freinsheim (DE); Friedrich, Holger, Dr., 67240 Bobenheim-Roxheim (DE); Zehner, Peter, Dr., 67071 Ludwigshafen (DE)

(56) Entgegenhaltungen:
- DE-C- 973 323
- US-A- 2 069 403

## Beschreibung

Die vorliegende Erfindung betrifft ein Verfahren zur katalytischen Herstellung von Alkalialkoholaten durch Umsetzung von Alkalimetallamalgam mit einem Alkohol in Gegenwart eines Katalysators.

Alkalialkoholate (systematischer Name: "Alkalialkanolate", auch "Alkalialkoxide" ist ein gebräuchliches Synonym) sind wohlbekannte Reagenzien in der organischen Chemie. Sie werden dort, wo starke Basen als Reaktionspartner benötigt werden, und als Katalysatoren bestimmter Reaktionen eingesetzt. In größeren Mengen hergestellt und verwendet werden praktisch nur aliphatische Alkalialkoholate des Lithiums, Natriums und Kaliums mit 1 bis 4 C-Atomen im Alkylrest des Alkohols, insbesondere Lithium-, Natriumund Kalium- -methylat, -ethylat, -n-propylat, -iso-propylat, -nbutylat und -tert.-butylat. Es sind eine Reihe von Verfahren zur Herstellung von Alkalialkoholaten bekannt. Die am weitesten verbreiteten sind die Umsetzung des Alkalimetalls mit dem Alkohol unter Wasserstoffentwicklung und die Umsetzung des Alkalimetallhydroxids mit dem Alkohol unter Abtrennung des als Nebenprodukt entstehenden Wassers. Ein Verfahren speziell zur Herstellung höherer Alkalialkoholate ist die Umsetzung eines Alkalimetallmethylats oder -ethylats mit einem höheren Alkohol unter Abtrennung von Methanol oder Ethanol. Dieses letztgenannte Verfahren und die Umsetzung von Alkalihydroxid mit Alkohol unter Abtrennung des Nebenprodukts Wasser ist der direkten Umsetzung von Alkalimetall mit Alkohol häufig aus wirtschaftlicher Sicht unterlegen, da sie vergleichsweise energieaufwendig sind.

Die direkte Umsetzung eines Alkalimetalls mit einem Alkohol ist die einfachste Herstellweise für Alkalialkoholate. Die Reaktivität der Alkalimetalle steigt in der Reihenfolge Lithium, Natrium, Kalium, Rubidium und Cäsium, und die Reaktivität der Alkohole sinkt mit dem Molekulargewicht des Alkohols und dem Verzweigungsgrad des Alkylrests. Die Umsetzung wird vorteilhafterweise mit einer Dispersion des Alkalimetalls in einem inerten Lösungsmittel oder mit Alkaliamalgam durchgeführt. Einen allgemeinen Überblick über aliphatische Alkalialkoholate, ihre Herstellung und Verwendung gibt beispielsweise Ullmann's Encyclopedia of Industrial Chemistry, Fifth Edition, Band A1, Weinheim 1985, als Punkt 3. unter dem Stichwort "Alcohols, Aliphatic". Einen Überblick über die technische Herstellung von Alkalialkoholaten aus Alkaliamalgam und dazu verwendete Reaktionsapparate gibt z. B: R. B. Mac-Mullin in: "By-Products of Amalgam-type Chlorine Cell", Chemical Engineering Progress Sept. 1950, S. 440-455, 448.

Bei der Herstellung höherer Alkalialkoholate - im Rahmen dieser Erfindung werden darunter Alkoholate mit mindestens 3 Kohlenstoffatomen im organischen Rest verstanden - ist die von MacMullin, l.c., beschriebene Vorgehensweise zwar anwendbar, aber aufgrund niedriger Reaktionsgeschwindigkeiten häufig wirtschaftlich nicht befriedigend. Es wurden daher Verfahren entwickelt, die durch Einsatz eines Katalysators eine höhere Raumzeitausbeute ermöglichen.

Derartige katalytische Verfahren sind altbekannt. So lehrt US-A-2,069,403 ein Verfahren zur Herstellung von Alkalimetallalkoholaten durch Umsetzung von Alkalimetallamalgam mit Alkoholen mit bis zu 4 Kohlenstoffatomen, bei dem die Umsetzung in Gegenwart eines Katalyators aus Graphit oder Eisen-Chrom-Legierungen, die wahlweise auch weitere Legierungsbestandteile wie Nickel, Molybdän, Wolfram, Mangan enthalten können, durchgeführt wird. Eine spezielle Ausführungsform dieses Verfahrens sowie ein typischer Zersetzer (Reaktoren zur Umsetzung von Amalgam mit Alkohol oder Wasser werden üblicherweise "Zersetzer" genannt) werden in US-A-2,336,045 gelehrt. Dabei werden das Amalgam und der Alkohol im Gegenstrom durch den Zersetzer geführt, der den Katalysator in Form einer Packung enthält. In diesen Schriften wird der Katalysator als "Elektrode" bezeichnet.

Im Verfahren von US-A-2,761,880 wird das Alkaliamalgam in Form einer Dispersion im Gegenstrom zum Alkohol eingesetzt, wobei zusätzlich Elektrodengraphit, Aktivkohle und/oder Eisenspäne als Katalysator verwendet werden. Ein bevorzugter Katalysator ist eine Mischung von Aktivkohle mit 10 - 20 % Eisenspänen.
DE-A-973 323 offenbart einen Katalysator, der 0,1 - 10 Gew.-% eines Metalls der Eisengruppe, insbesondere Eisen oder Nickel, auf einem Graphitträger enthält. EP-A-177 768 lehrt einen Katalysator zur Alkalimetallalkoholatherstellung, der aus Schwermetalloxid oder -oxiden, insbesondere einer Mischung aus Nickel- und Molybdänoxid, die auf die Oberfläche eines stückigen Anthrazitträgers aufgebracht sind, besteht. US-A-5,262,133 lehrt die Verwendung von Wolframcarbid, Eisen auf Kohlenstoffträger, Iridium, Ruthenium oder deren Mischungen als Katalysator. EP-A-810 193 offenbart Katalysatoren aus Carbiden und Nitriden von Chrom, Molybdän oder Wolfram, sowie aus Titancarbid. Im Verfahren von DE-A-198 02 013 werden Katalysatoren aus Übergangsmetallcarbiden, -nitriden oder -carbonitriden, insbesondere von Molybdän- oder Wolframcarbid, in Pulverform verwendet, und im Verfahren von EP-A-1 018 499 wird dieser Pulverkatalysator durch Einwirkung von Ultraschall suspendiert.

In vielen bekannten Verfahren ist jedoch die Raumzeitausbeute noch unbefriedigend und in den bekannten Verfahren mit höherer Raumzeitausbeute werden aufgrund geringer Standzeit oder hohen Preises vergleichsweise teure Katalysatoren benötigt. Es bestand daher die Aufgabe, ein einfacheres, wirtschaftlich befriedigenderes Verfahren zu finden, das eine möglichst hohe Raumzeitausbeute ermöglicht und mit einem preiswerten Katalysator auskommt.

Demgemäß wurde ein Verfahren zur Herstellung von Alkalialkoholaten durch Umsetzung von Alkalimetallamalgam mit Alkohol in Gegenwart eines Katalysators gefunden, das dadurch gekennzeichnet ist, dass man einen Katalysator verwendet, der Eisen mit einem Kohlenstoffgehalt von mindestens 0,3 Gew.-% in Form einer Eisen-Kohlenstoff-Legierung enthält.

Der im erfindungsgemäßen Verfahren verwendete Katalysator ist preiswert, zeigt hohe Standzeiten und ermöglicht hohe Raumzeitausbeuten. Es ist ein besonderer Vorzug des erfindungsgemäßen Verfahrens, dass es problemlos in bestehenden Zersetzern durchgeführt werden kann, so dass keine Umrüstung bestehender Anlagen erforderlich ist.

Als Alkalimetall wird im erfindungsgemäßen Verfahren Lithium, Natrium, Kalium, Rubidium oder Cäsium verwendet. Vorzugsweise werden Natrium oder Kalium verwendet. Das Alkalimetallamalgam, das das verwendete Alkalimetall enthält, kann mit jedem bekannten Verfahren zur Herstellung von Alkaliamalgam erzeugt werden, beispielsweise durch Vermischen von Alkalimetall und Quecksilber, wird aber zumeist in bekannter Weise durch Elektrolyse einer Lösung eines entsprechenden Salzes, beispielsweise eines Halogenids, in der Regel das Alkalichlorid, in einer Elektrolysezelle hergestellt. Besonders dazu geeignet ist der Typ von Elektrolysezellen, in dem das bekannte Amalgamverfahren zur Herstellung von Chlor und Natronlauge üblicherweise durchgeführt wird. Das Amalgam enthält in der Regel mindestens 0,05 Gew.-% Alkalimetall, vorzugsweise mindestens 0,1 Gew.-% und in besonders bevorzugter Weise mindestens 0,2 Gew.-%. Es enthält in der Regel höchstens 1 Gew.-% Alkalimetall, vorzugsweise höchstens 0,7 Gew.-% und in besonders bevorzugter Weise höchstens 0,5 Gew.-%.

Als Alkohol kann im Prinzip jede Verbindung mit einer Hydroxigruppe an einem kohlenstoffhaltigen Rest eingesetzt werden. Im allgemeinen werden aliphatische Alkohole eingesetzt, insbesondere solche mit einem geradkettigen oder verzweigten Kohlenstoffrest mit einem bis 8 Kohlenstoffatomen. In bevorzugter Weise werden primäre, sekundäre oder tertiäre Alkohole mit einem bis 5 Kohlenstoffatomen eingesetzt, und ein besonders bevorzugter Weise solche mit einem bis vier Kohlenstoffatomen. Beispiele für im erfindungsgemäßen Verfahren eingesetzte Alkohole sind Methanol, Ethanol, n-Propanol, iso-Propanol, n-Butanol, 2-Butanol (sec.-Butanol) 2-Methyl-1-propanol (iso-Butanol), 2-Methyl-2-propanol (tert.-Butanol), 1-, 2- oder 3-Pentanol, neo-Pentanol, tert.-Pentanol, Hexan-, Heptan- oder Octanol.

Wirtschaftlich am bedeutendsten und daher bevorzugte Produkte des erfindungsgemäßen Verfahrens sind Natrium- und Kalium- -methylat, -ethylat und -tert.-butylat. Bevorzugt ist daher die Verwendung von Methanol, Ethanol oder tert.-Butanol.

Es ist ebenso möglich, Alkohole mit mehr als einer Hydroxigruppe zu verwenden, beispielsweise Glykol.

Nachdem die Alkalialkoholate durch Substitution des Wasserstoffatoms der Hydroxigruppe des Alkohols durch das Alkalimetall gebildet werden, findet sich der Alkylrest des verwendeten Alkohols direkt im Alkalialkoholat wieder.

Das erfindungsgemäße Verfahren ist insbesondere geeignet zur Herstellung von Natriummethanolat, Kaliummethanolat, Natriumethanolat, Kaliumethanolat, Natrium-tert.-butylat und Kalium-tert.-butylat.

Der Katalysator enthält Eisen mit einem Kohlenstoffgehalt von mindestens 0,3 Gew.-%. In bevorzugter Weise enthält das Eisen mindestens 0,5 Gew.-% Kohlenstoff und in besonders bevorzugter Weise mindestens 1 Gew.-% Kohlenstoff. Im allgemeinen beträgt der Kohlenstoffgehalt des Eisens höchstens 4 Gew.-%. Der Katalysator ist keine Mischung aus makroskopischen Eisenteilchen und makroskopischen Kohlenstoffteilchen, sondern eine Eisen-Kohlenstoff-Legierung, enthält also fest in das Gefüge eingebauten Kohlenstoff, beispielsweise in Form einer festen Lösung oder als im metallischen Gefüge homogen ausgeschiedenen Kohlenstoff. Ein über 4 Gew.-% hinausgehender Kohlenstoffgehalt liegt im allgemeinen nicht mehr im Eisen gelöst oder homogen im Gefüge ausgeschieden vor, sondern in Form von größeren, losen oder inhomogen verteilten Kohlenstoffpartikeln. Diese beeinflussen das Verfahren nicht wesentlich.

Neben Kohlenstoff können im Eisen weitere Bestandteile enthalten sein, beispielsweise typische Legierungselemente oder Reste von Zuschlagstoffen bei der Eisen- oder Stahlherstellung wie Silicium, Schwefel, Chrom, Mangan, Molybdän und/oder Phosphor. Wird Carbonyleisen verwendet, können herstellungsbedingt auch Sauerstoff und/oder Stickstoff enthalten sein. Vorzugsweise enthält das im erfindungsgemäßen Verfahren verwendete kohlenstoffhaltige Eisen über die aus der Eisengewinnung herrührenden unvermeidlichen Verunreinigungen (beispielsweise Reste der dort üblicherweise verwendeten Zuschlagstoffe oder Sauerstoff und/oder Stickstoff bei Carbonyleisen) hinaus keine absichtlich zugesetzten weiteren Bestandteile.

Der Katalysator kann in seiner Aktivmasse neben dem kohlenstoffhaltigen Eisen weitere, seine katalytischen Eigenschaften chemisch beeinflussende Bestandteile enthalten, beispielsweise Promotoren oder andere bekannte katalytisch aktive Komponenten. Vorzugsweise wird ein Katalysator verwendet, dessen Aktivmasse solche Nebenkomponenten nur in untergeordnetem Umfang enthält, also im wesentlichen aus Eisen, das mindestens 0,3 Gew.-% Kohlenstoff enthält, besteht. In besonders bevorzugter Weise wird ein Katalysator verwendet, dessen Aktivmasse aus Eisen, das mindestens 0,3 Gew.-% Kohlenstoff enthält, besteht, und nur unvermeidliche Verunreinigungen enthält.

Die Aktivmasse kann auf einem Katalysatorträger aufgebracht sein, beispielsweise auf Kohlenstoff, insbesondere auf Graphit oder Anthrazit, wird jedoch vorzugsweise als trägerfreier Katalysator verwendet.

In bevorzugter Weise wird ein Katalysator verwendet, der im wesentlichen aus kohlenstoffhaltigem Eisen mit einem Kohlenstoffgehalt von mindestens 0,3 Gew.-% besteht. In besonders bevorzugter Weise wird ein Katalysator verwendet, der aus kohlenstoffhaltigem Eisen mit einem Kohlenstoffgehalt von mindestens 0,3 Gew.-% besteht und über die aus der Eisengewinnung herrührenden unvermeidlichen Verunreinigungen hinaus keine absichtlich zugesetzten Mengen weiterer Bestandteile enthält. Typische Zusammensetzungen sind beispielsweise 3,8 Gew.-% Kohlenstoff, 2,1 Gew.-% Silicium, Spuren von Schwefel, Chrom, Mangan, Molybdän und Phosphor, Rest Eisen, oder 3,3 Gew.-% Kohlenstoff, 2,5 Gew.-% Silicium, Spuren von Schwefel, Chrom, Mangan, Molybdän und Phosphor, Rest Eisen, oder 1,8 Gew.-% Kohlenstoff, 1,7 Gew.-% Stickstoff, 0,2 Gew.-% Sauerstoff, Rest Eisen.

Ein im erfindungsgemäßen Verfahren bevorzugt verwendeter Katalysator ist Gusseisen, gießfähiges kohlenstoffhaltiges Eisen mit einem Kohlenstoffgehalt von 2 bis 4 Gew.-%, insbesondere der sogenannte Grauguss, der einen aus Silikatzuschlag herrührenden Siliciumgehalt von ca. 2 bis 3 Gew.-% aufweist, und bei dem durch langsames Abkühlen des zunächst flüssigen Roheisens Kohlenstoff, etwa als sogenannter Lamellen- oder Kugelgraphit im Gefüge ausgeschieden wird, so dass Bruchflächen grau erscheinen (zur Definition der Begriffe siehe Römpp Chemie Lexikon, Thieme Verlag, Stuttgart 1990, Band 2, ISBN 3-13-734709-2, Stichwort "Gußeisen"). Herstellung und Verarbeitung von Gusseisen sind wohlbekannt.

Ein im erfindungsgemäßen Verfahren ebenso bevorzugt verwendeter Katalysator ist Carbonyleisen mit einem Kohlenstoffgehalt von mindestens 0,3 Gew.-%, insbesondere von mindestens 1 Gew.-%. Carbonyleisen ist durch Zersetzung von Eisencarbonyl hergestelltes Eisen, dessen Kohlenstoff- Stickstoff- und Sauerstoffgehalt in bekannter Weise durch entsprechende Parameterwahl bei der Zersetzung und/oder durch Nachbehandlung eingestellt werden kann (siehe z. B. Ullmann's Encyclopedia of Industrial Chemistry, Fifth Edition, Band A14, Weinheim 1989, als Punkt 3.5. Iron Pentacarbonyl/Uses unter dem Stichwort "Iron Compounds", und die dort zitierte Literatur). Herstellung und Verarbeitung von Carbonyleisen sind wohlbekannt.

Die katalytische Wirkung des Katalysators kann durch eine vor seinem Einsatz durchgeführte Behandlung mit Mineralsäure, beispielsweise Schwefelsäure, Salpetersäure oder Salzsäure, vorzugsweise Salzsäure oder Salpetersäure, noch gesteigert werden. Dazu wird der Katalysator mit der Säure in Kontakt gebracht. Die Konzentration der Säure beträgt im allgemeinen mindestens 1 Gew.-%, vorzugsweise mindestens 3 Gew.-% und in besonders bevorzugter Weise mindestens 5 Gew.-%. Die Konzentration beträgt im allgemeinen höchstens 30 Gew.-%, vorzugsweise höchstens 20 Gew.-% und in besonders bevorzugter Weise höchstens 15 Gew.-%. Besonders geeignet sind 10 Gew.-%-ige Salzsäure oder 10 Gew.-%-ige Salpetersäure. Die Säurebehandlung des Katalyators dauert im allgemeinen mindestens eine Minute, vorzugsweise mindestens 5 Minuten und in besonders bevorzugter Weise mindestens 10 Minuten. Sie ist im allgemeinen nach höchstens zwei Stunden abgeschlossen, vorzugsweise nach höchstens 90 Minuten und in besonders bevorzugter Weise nach höchstens einer Stunde. Die Temperatur beträgt im allgemeinen mindestens 0 °C, vorzugsweise mindestens 10 °C und in besonders bevorzugter Weise mindestens 20 °C. Sie beträgt ferner im allgemeinen höchstens 100 °C, vorzugsweise höchstens 60 °C und in besonders bevorzugter Weise höchstens 40 °C. Der Katalysator wird dazu in einem Reaktionsgefäß, beispielsweise dem Zersetzer, mit der Säure überschichtet. Die Säure kann wahlweise auch durch das Reaktionsgefäß bewegt werden. Bei dieser Behandlung entsteht üblicherweise etwas Wasserstoff, der wie bei der anschließenden Umsetzung selbst abzuführen ist. Wenn gewünscht, wird der Katalysator nach der Säurebehandlung mit Wasser gespült. Diese Säurebehandlung ist auch zur Reaktivierung desaktivierter Katalysatoren geeignet.

Der Katalysator wird als Pulver oder vorzugsweise in stückiger Form eingesetzt. Partikelgrößen mit Abmessungen in den drei Raumrichtungen von mindestens 0,5 mm, vorzugsweise 1 mm, und höchstens 20 mm, vorzugsweise höchstens 10 mm, sind in aller Regel gut zur Verwendung in gängigen Zersetzern geeignet. Um die Bildung regelmäßiger räumlicher Strukturen zu vermeiden, werden vorzugsweise unregelmäßig geformte Partikel eingesetzt. Diese können durch Brechen oder Zerspanen aus Massivmaterial, beispielsweise handelsüblichem Gusseisen, hergestellt werden. Besonders bequem ist die Verwendung von Kleineisenwaren aus Gusseisen als Katalysator, beispielsweise Gusseisen-Schrauben oder -Gewinderingen. Feinteiliges Eisen, wie beispielsweise handelsübliches Carbonyleisenpulver, kann durch jedes bekannte Formgebungsverfahren zu Katalysatorpartikeln der gewünschten Größe verformt werden, beispielsweise durch Pressen und/oder Sintern, sowie gegebenenfalls Brechen oder Zerspanen eines so hergestellten größeren Formkörpers.

Der Katalysator wird in einem üblichen Zersetzer eingesetzt.

Zur Durchführung des erfindungsgemäßen Verfahrens werden im mit Katalysator gefüllten Zersetzer das Alkalimetallamalgam und der Alkohol umgesetzt. Üblicherweise wird der Alkohol im Überschuss eingesetzt, so dass eine Lösung des Alkalialkoholats im entsprechenden Alkohol hergestellt wird, da die Abtrennung der in Reinsubstanz bei Umgebungstemperatur festen Alkalialkoholate vom Quecksilber oder - bei unvollständiger Umsetzung des Alkalimetalls - alkaliärmeren Amalgam ansonsten nur umständlich durchzuführen wäre. Das Verfahren kann diskontinuierlich oder kontinuierlich durchgeführt werden, die kontinuierliche Verfahrensweise ist in aller Regel wirtschaftlicher. Typischerweise werden dazu der Alkohol und das Amalgam kontinuierlich im Gegenstrom durch den mit Katalysator gefüllten Zersetzer geleitet.

Das den Zersetzer verlassende Quecksilber wird wieder in die Alkalimetallamalgamherstellung zurückgeführt. Bei unvollständiger Umsetzung des Alkalimetalls kann das alkaliärmere Amalgam ebenso wieder in die Alkaliamalgamherstellung zurückgeführt werden, es kann wahlweise jedoch auch in den Reaktor zurückgeführt werden, bis der gewünschte Alkalimetallumsatz erreicht ist. Es ist ebenso möglich, den Zersetzer verlassendes alkaliärmeres Amalgam mit einem anderen Alkohol zu einem Alkoholat und Quecksilber oder mit Wasser zu Alkalihydroxid und Quecksilber umzusetzen.

Die den Zersetzer verlassende Alkalialkoholatlösung wird entweder direkt verwendet, oder das enthaltene Alkalialkoholat wird mittels üblicher Methoden isoliert (beispielsweise durch Eindampfen der Lösung und wahlweise anschließende Reinigung, beispielsweise durch Umkristallisation), oder sie wird in den Zersetzer zurückgeführt, bis die gewünschte Konzentration an Alkalialkoholat erreicht wird. Vorzugsweise wird der Alkohol-Mengenstrom durch den Reaktor so eingestellt, dass eine Alkoholatlösung der gewünschten Konzentration ohne Rückführung aus dem Zersetzer entnommen wird.

Die Reaktionstemperatur beträgt im allgemeinen mindestens 0 °C, vorzugsweise mindestens 10 °C und in besonders bevorzugter Weise mindestens 20 °C. Da sich das Alkalialkoholat bei zu hohen Temperaturen zersetzen kann, kann die Reaktionstemperatur nicht beliebig hoch gewählt werden, ihre Obergrenze ist die Temperatur, bei der durch Zersetzung in wirtschaftlich nicht mehr tolerierbarer Menge Produktverlust eintritt. Häufig und in bevorzugter Weise wird die Reaktion bei der Siedetemperatur des verwendeten Alkohols durchgeführt.

Unter Umständen, vor allem bei der Umsetzung reaktiverer Alkohole (deren Reaktivität sinkt bekanntlich mit dem Molekulargewicht des Alkohols und mit dem Verzweigungsgrad des Substituenten) ist die Abführung der Reaktionswärme erforderlich, was durch übliche Kühlvorrichtungen erfolgt. Die Reaktionswärme wird beispielsweise durch Kühlschlangen im oder am Reaktor, Führung der den Reaktor verlassenden Stoffströme durch Wärmetauscher, oder durch Siedeoder Rückflußkühlung durch absiedenden Alkohol abgeführt. Der als Nebenprodukt entstehende Wasserstoff wird gasförmig aus dem Zersetzer abgeleitet, er wird entsorgt, beispielsweise durch Verbrennung, oder vorzugsweise verwertet, beispielsweise als Hydrierwasserstoff für Hydrierreaktionen.

Die Katalysatormenge (d.h., auch die Zersetzergröße), die Verweilzeit der Reaktanden im Reaktor und die Reaktionstemperatur werden so gewählt, dass eine etwaige Zersetzung von Einsatzstoffen oder Produkten vermieden wird oder in wirtschaftlich tolerierbarem Rahmen bleibt.

### Beispiele

### Versuchsdurchführung

In einer mit Argon gespülten Rührapparatur wurden 2000 g Quecksilber mit einem Kaliumgehalt von 0,3 Gew.-% mit 200 g tert.-Butanol in Gegenwart eines Katalyators umgesetzt. Die Reaktionsmischung wurde gerührt (300 Umdrehungen pro Minute), die Reaktionswärme wurde durch Kondensation absiedenden tert.-Butanols mittels eines Rückflußkühlers abgeführt. Anschließend wurden die Umsätze und Ausbeuten bestimmt. Die Ergebnisse der Versuche sind in Tabelle 1 zusammengefasst.

### Beispiel 1

Als Katalysator wurden 20 g kohlenstoffhaltiges Eisen (3,8 Gew.-% Kohlenstoff, 2,1 Gew.-% Silicium, Spuren von Schwefel, Chrom, Mangan, Molybdän und Phosphor, Rest Eisen, Korngröße 0 - 3 mm) eingesetzt.

### Beispiel 2

Als Katalysator wurden 100 g Gusseisen-Gewinderinge (5,5 * 7 mm) eingesetzt.

### Beispiel 3

Als Katalysator wurden 100 g gesintertes und anschließend gebrochenes Carbonyleisen (Korngröße 2 - 4 mm) mit einem Kohlenstoffgehalt von 1,4 Gew.-% eingesetzt.

### Vergleichsbeispiel V1

Als Katalysator wurden 20 g Molybdäncarbid (Korngröße ca. 2 µm) eingesetzt.

### Vergleichsbeispiel V2

Als Katalysator wurden 20 g Elektrodengraphit (0 - 40 µm) eingesetzt.

### Vergleichsbeispiel V3

Als Katalysator wurden zunächst 50 g Eisen (durch thermische Zersetzung von Eisenpentacarbonyl hergestelltes Eisenpulver mit einem Kohlenstoffgehalt von unter 0,2 Gew.-%, Partikelgröße > 500 µm, sogenanntes "Überkorn" aus der technischen Carbonyleisenherstellung) verwendet. Nach 8,3 Stunden Reaktionsdauer wurden nochmals 150 g dieses Katalysators zugegeben, und nach insgesamt 16,8 Stunden Reaktionsdauer zusätzlich 20 g Elektrodengraphit.

**Tabelle 1**

| Beispiel | 1 | 2 | 3 | V1 | V2 | V3 | | |
|---|---|---|---|---|---|---|---|---|
| Reaktionsdauer [Stunden] | 9 | 14 | 14 | 10 | 13 | 8,3 | 16,8 | 25,8 |
| Kaliumumsatz [Mol.-%] | 100 | 80 | 79 | 100 | 40 | 3 | 5,3 | 9,4 |
| Ausbeute [Mol.-%] | 100 | 80 | 79 | 100 | 40 | 3 | 5,1 | 8,6 |

Beispiel 1 zeigt, dass mit dem erfindungsgemäß verwendeten Katalysator in kürzerer Reaktionsdauer die gleichen Ausbeuten erreicht werden wie mit dem teuren Molybdäncarbid (Vergleichsbeispiel V1). Selbst der besonders bequeme und preiswerte Katalysator Gusseisen-Gewinderinge ist deutlich aktiver (höherer Umsatz bei vergleichbarer Reaktionsdauer) als der bekannte Katalysator Elektrodengraphit (Vergleichsbeispiel V2). Das gleiche gilt für das Carbonyleisen von Versuch 3. Vergleichsbeispiel V3 zeigt, dass die Verwendung von Reineisen oder einer makroskopischen Mischung aus Reineisen und Kohlenstoff bei weitem nicht die katalytische Wirkung des erfindungsgemäß verwendeten kohlenstoffhaltigen Eisens zeigt.

## Patentansprüche

1. Verfahren zur Herstellung von Alkalialkoholaten durch Umsetzung von Alkalimetallamalgam mit Alkohol in Gegenwart eines Katalysators, **dadurch gekennzeichnet, dass** man einen Katalysator verwendet, der Eisen mit einem Kohlenstoffgehalt von mindestens 0,3 Gew.-% in Form einer Eisen-Kohlenstoff-Legierung enthält.

2. Verfahren nach Anspruch 1, **dadurch gekennzeichnet, dass** der Katalysator im wesentlichen aus Eisen mit einem Kohlenstoffgehalt von mindestens 0,3 Gew.-% in Form einer Eisen-Kohlenstoff-Legierung besteht.

3. Verfahren nach Anspruch 2, **dadurch gekennzeichnet, dass** der Katalysator aus Eisen mit einem Kohlenstoffgehalt von mindestens 0,3 Gew.-% in Form einer Eisen-Kohlenstoff-Legierung besteht, das außer unvermeidlichen Verunreinigungen keine weiteren Bestandteile enthält.

4. Verfahren nach Anspruch 3, **dadurch gekennzeichnet, dass** der Katalysator Carbonyleisen ist.

5. Verfahren nach Anspruch 3, **dadurch gekennzeichnet, dass** der Katalysator Gusseisen ist.

6. Verfahren nach Anspruch 1, **dadurch gekennzeichnet, dass** man den Katalysator vor seinem Einsatz mit Mineralsäure behandelt.

7. Verfahren nach den Ansprüchen 1 bis 6, **dadurch gekennzeichnet, dass** man einen aliphatischen primären, sekundären oder tertiären Alkohol mit einem bis 8 Kohlenstoffatomen im Alkylrest verwendet.

8. Verfahren nach Anspruch 7, **dadurch gekennzeichnet, dass** man Methanol, Ethanol oder tert.-Butanol verwendet.

9. Verfahren nach Anspruch 7, **dadurch gekennzeichnet, dass** man als Alkalimetall Natrium oder Kalium einsetzt.

10. Verfahren nach Anspruch 8, **dadurch gekennzeichnet, dass** man als Alkalimetall Natrium oder Kalium einsetzt.

## Claims

1. A process for preparing alkali metal alkoxides by reacting alkali metal amalgam with alcohol in the presence of a catalyst comprising iron having a carbon content of at least 0.3% by weight in the form of an iron-carbon alloy.

2. A process as claimed in claim 1, wherein the catalyst consists essentially of iron having a carbon content of at least 0.3% by weight in the form of an iron-carbon alloy.

3. A process as claimed in claim 2, wherein the catalyst consists of iron having a carbon content of at least 0.3% by weight and contains no further constituents apart from unavoidable impurities in the form of an iron-carbon alloy.

4. A process as claimed in claim 3, wherein the catalyst is carbonyl iron.

5. A process as claimed in claim 3, wherein the catalyst is cast iron.

6. A process as claimed in claim 1, wherein the catalyst is treated with mineral acid before use.

7. A process as claimed in any of claims 1 to 6, wherein an aliphatic primary, secondary or tertiary alcohol having from 1 to 8 carbon atoms in the alkyl radical is used.

8. A process as claimed in claim 7, wherein methanol, ethanol or tert-butanol is used.

9. A process as claimed in claim 7, wherein sodium or potassium is used as alkali metal.

10. A process as claimed in claim 8, wherein sodium or potassium is used as alkali metal.

## Revendications

1. Procédé de préparation d'alcoolates de métal alcalin par réaction d'amalgame de métal alcalin avec de l'alcool en présence d'un catalyseur, **caractérisé en ce qu'**on utilise un catalyseur qui contient du fer présentant une teneur en carbone d'au moins 0,3% en poids sous la forme d'un alliage de fer-carbone.

2. Procédé suivant la revendication 1, **caractérisé en ce que** le catalyseur est constitué essentiellement de fer présentant une teneur en carbone d'au moins 0,3% en poids sous la forme d'un alliage de fer-carbone.

3. Procédé suivant la revendication 2, **caractérisé en ce que** le catalyseur est constitué de fer présentant une teneur en carbone d'au moins 0,3% en poids sous la forme d'un alliage de fer-carbone qui, en dehors d'impuretés inévitables, ne contient pas d'autres éléments.

4. Procédé suivant la revendication 3, **caractérisé en ce que** le catalyseur est du fer-carbonyle.

5. Procédé suivant la revendication 3, **caractérisé en ce que** le catalyseur est de la fonte de fer.

6. Procédé suivant la revendication 1, **caractérisé en ce que**, avant sa mise en oeuvre, on traite le catalyseur avec de l'acide minéral.

7. Procédé suivant l'une des revendications 1 à 6, **caractérisé en ce qu'**on utilise un alcool aliphatique primaire, secondaire ou tertiaire comportant 1 à 8 atomes de carbone dans le radical alkyle.

8. Procédé suivant la revendication 7, **caractérisé en ce qu'**on utilise du méthanol, de l'éthanol ou du butanol tertiaire.

9. procédé suivant la revendication 7, **caractérisé en ce qu'**on met en oeuvre du sodium ou du potassium comme métal alcalin.

10. Procédé suivant la revendication 8, **caractérisé en ce qu'**on met en oeuvre du sodium ou du potassium comme métal alcalin.
